# EUROPEAN PATENT APPLICATION

(11) **EP 3 184 030 A1**
(43) Date of publication of application: **28.06.2017**
(21) Application number: 14900124.0
(22) Date of filing: 22.08.2014
(51) Int. Cl.: A61B 5/00, A61B 10/00, G01N 22/04

(54) **STATE ESTIMATING METHOD, STATE ESTIMATING SYSTEM, CLOTHING, AND MONITORING SYSTEM**

(71) Applicant: Fujitsu Limited, Kawasaki-shi, Kanagawa 211-8588 (JP)
(72) Inventor: YAMAJI, Takayuki, Kawasaki-shi, Kanagawa 211-8588 (JP)
(74) Representative: Hoffmann Eitle
(86) International application number: PCT/JP2014/071965
(87) International publication number: WO 2016/027359

(57) **Abstract**

A method of determining a state is disclosed. The method is to be executed by a computer The method includes determining a moisture state according to a reception state of a reflection wave of a radio wave, the radio wave being transmitted to a human body via a wear item, the wear item being on the human body, and outputting according to the determined result.

## Description

### [Technical Field]

The present invention is related to a method of determining a state, a state determination system, a wear item, and a monitoring system.

### [Background Art]

A moisture meter for measuring moisture of a test subject is known, which moisture meter includes a moisture measurement part of a probe type configured to be held under the armpit of the test subject to measure the moisture under the armpit for measuring the moisture content of the test subject (see Patent Document 1, for example). According to the moisture meter, the moisture measurement part transmits light from a light emitting part onto the moisture on skin under the armpit, receives reflected light thereof at a light reception part, and measures the moisture content according to a change in the received light amount.

### [Citation List]

[Patent Document 1] Japanese Laid-open Patent Publication No. 2012-71056
[Patent Document 2] Japanese Laid-open Patent Publication No. 2014-039666

### [Summary]

### [Technical Problem]

However, according to the configuration disclosed in Patent Document 1, since the moisture meter is of a contact type such that the moisture meter is held under the armpit, sweat, oil or the like tends to adhere to the light emitting part or the light reception part, which leads to the reduced accuracy of measurement. Further, in the case of the measurement with the light, a light absorption amount varies according to the color of the reflection surface (i.e., the skin). For example, the color of the skin is not uniform due to lentigo, stain, a part of blood vessels, etc. Further, in the case of the measurement with the light, the light absorption factor varies according to materials of wear items, and thus the amount of the reflected light varies according to the material of the wear item.

Accordingly, it is an object of one aspect of the invention to provide a method of determining a state, etc., that can output a moisture state with increased accuracy.

### [Solution to Problem]

According to an aspect of the disclosure, a method of determining a state is provided, the method being to be executed by a computer, said method comprising: determining a moisture state according to a reception state of a reflection wave of a radio wave, the radio wave being transmitted to a human body via a wear item, the wear item being on the human body, and outputting according to the determined result.

### [Advantageous Effects of Invention]

A method of determining a state, etc., that can output a moisture state with increased accuracy can be obtained.

### [Brief Description of Drawings]

Fig. 1 is a diagram illustrating an example of a radio wave transmission part 1 and a radio wave reception part 2.
Fig. 2 is a block diagram illustrating an example of a hardware configuration of a state determination system according to an embodiment.
Fig. 3 is a block diagram illustrating an example of a radio wave transmission/reception part.
Fig. 4 is a block diagram illustrating an example of a functional configuration of a state determination system 10-1.
Fig. 5 is a diagram illustrating a relationship between absolute humidity and an amplitude of a waveform of a Doppler sensor output.
Fig. 6 is a diagram explaining a correlation between an amplitude of a reflection wave and a sweating amount.
Fig. 7 is a flowchart illustrating an example of a state determination process executed by a CPU 11 of the state determination system 10-1.
Fig. 8 is a block diagram illustrating an example of a hardware configuration of a state determination system according to another embodiment.
Fig. 9 is a block diagram illustrating an example of a functional configuration of a state determination system 10-2 according to an example.
Fig. 10 is a diagram illustrating an example of logic for determining a hazard level of a heat stroke.
Fig. 11 is a flowchart illustrating an example of a state determination process executed by a CPU 11 of the state determination system 10-2.
Fig. 12 is a diagram illustrating an example of a way of holding a radio wave transmission/reception part 25.
Fig. 13 is a diagram schematically illustrating an example of a hardness 7.
Fig. 14 is a diagram illustrating an example of a configuration of a monitoring system 110.

### [Description of Embodiments]

In the following, embodiments are described in detail with reference to appended drawings.

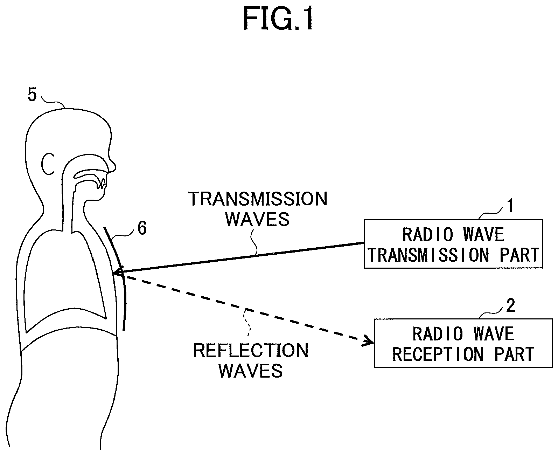

Fig. 1 is a diagram illustrating an example of a radio wave transmission part 1 and a radio wave reception part 2. A state determination system 10-1 includes a radio wave transmission part 1 and a radio wave reception part 2. It is noted that, in the following, a person whose moisture state including sweating state is to be determined is also referred to as "a test subject".

The radio wave transmission part 1 emits radio waves to a human body of a test subject 5 via a wear item 6 on the test subject 5. A band of a frequency of the radio wave to be used is arbitrary; however, such a frequency band is desired in which the radio waves are not harmful for the human body and difficult to be absorbed in the moisture. An example of the radio waves may be UHF(Ultra High Frequency) waves or SHF(Super High Frequency) waves. Further, the radio waves may have frequency in 2.4 GHz band, for example. The wear item 6 may be of any type of items that are formed of any material and can be on the human body. The wear item 6 may include clothes, a diaper, etc. The expression "via the wear item 6" means that there is the wear item 6 between a portion (chest, for example) of the human body on which the radio waves impinge and the radio wave transmission part 1. It is noted that, in the example illustrated in Fig. 1, the wear item 6 is an underwear item that is used such that the wear item 6 is on an upper-body of the test subject 5. The radio wave reception part 2 receives the reflection waves of the radio waves from the test subject 5 via the wear item 6.

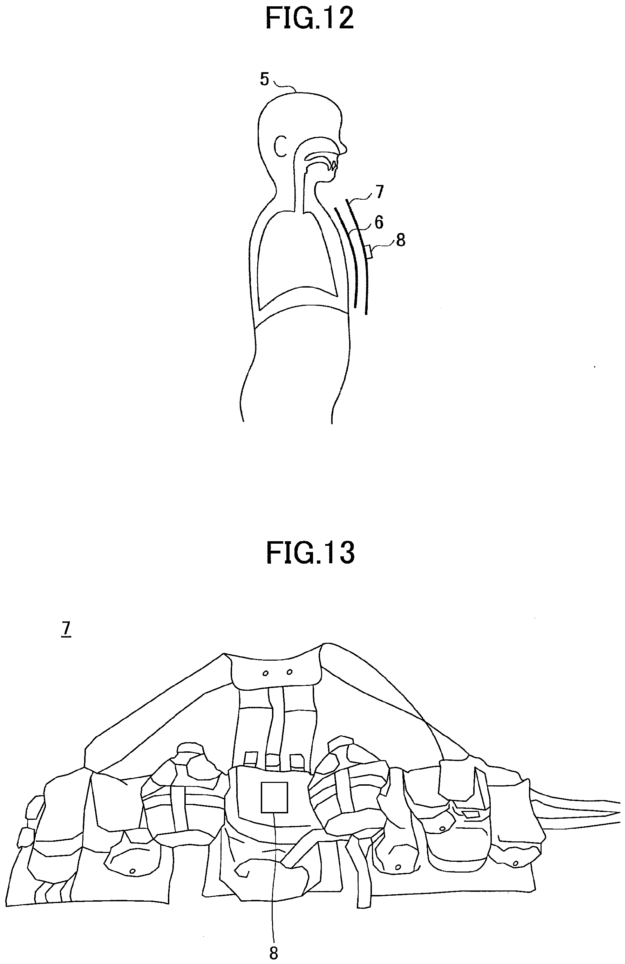

The radio wave transmission part 1 and the radio wave reception part 2 of the state determination system 10-1 may be attached to an outer surface of the wear item 6 that is closest to the human body, or may be attached to another outer wear item (not illustrated) that is on the wear item 6 (see Fig. 12). For example, the radio wave transmission part 1 and the radio wave reception part 2 may be attached to a wear item called "a hardness" (see a hardness 7 in Fig. 13) that is used for training session of troops, etc.

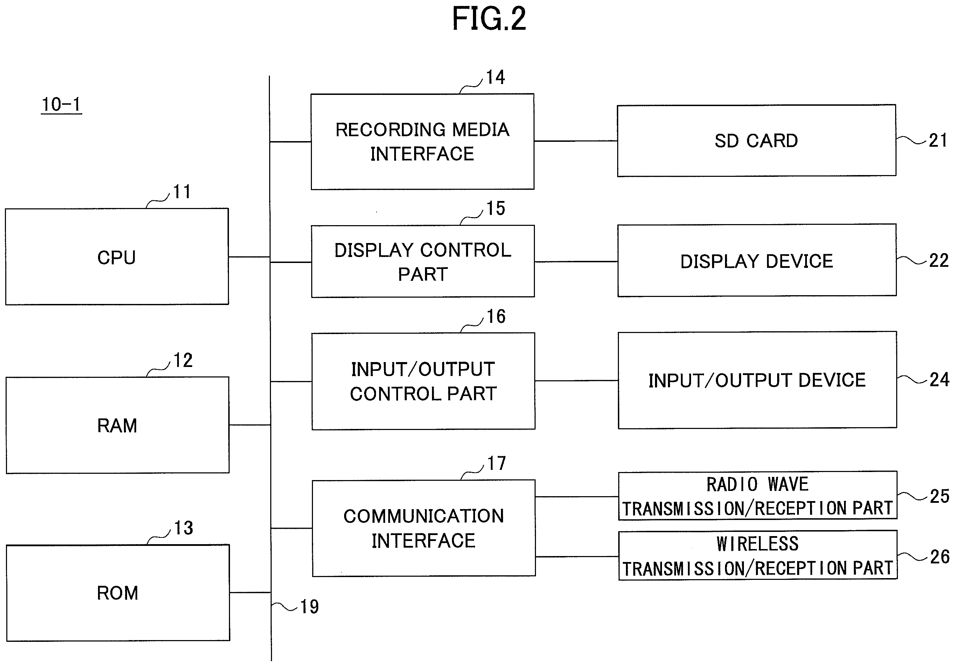

Fig. 2 is a block diagram illustrating an example of a hardware configuration of a state determination system 10-1 according to an embodiment.

The state determination system 10-1 includes a CPU (Central Processing Unit) 11, a RAM (Random Access Memory) 12, a ROM (Read Only Memory) 13, a recording media interface 14, and a display control part 15, coupled to each other via a bus 19, as illustrated in Fig. 2. Further, the state determination system 10-1 includes an input/output control part 16 and a communication interface 17. The recording media interface 14 can be coupled to a recording medium such as a SD (Secure Digital) card (or a memory card) 21, etc. The display control part 15 is coupled to a display device 22. The input/output control part 16 is coupled to an input/output device 24. The input/output device 24 may be a touch panel, a speaker, etc. The functions of the display device 22 and the input/output device 24 may be implemented by the touch panel. It is noted that, the recording media interface 14, the SD card 21, the input/output control part 16 and the input/output device 24, the display device 22 and the display control part 15, and/or a wireless transmission/reception part 26 may be omitted, if appropriate.

The communication interface 17 is coupled to a radio wave transmission/reception part 25 and the wireless transmission/reception part 26. The radio wave transmission/reception part 25 includes the radio wave transmission part 1 and the radio wave reception part 2 illustrated in Fig. 1. The wireless transmission/reception part 26 includes a transmission/reception part that is capable of communicating via a wireless communication network used by cellular phones. The wireless transmission/reception part 26 may include a Near Field Communication (NFC) part, a Bluetooth (trademark) communication part, a Wi-Fi (Wireless-Fidelity) transmission/reception part, an infrared transmission/reception part, etc.

The CPU 11 has a function of controlling operations of the state determination system 10-1 as a whole. The RAM 12 and the ROM 13 form a storage part in which programs to be executed by the CPU 11 or items of data are stored. The programs includes one or more programs that cause the CPU 11 to execute a state determination process to function as a state determination system. The storage part may include the SD card 21. The storage part that stores the programs is an example of a computer-readable recording medium.

The display device 22 has a function of displaying operation screens, etc., as a result of the state determination process under control of the display control part 15.

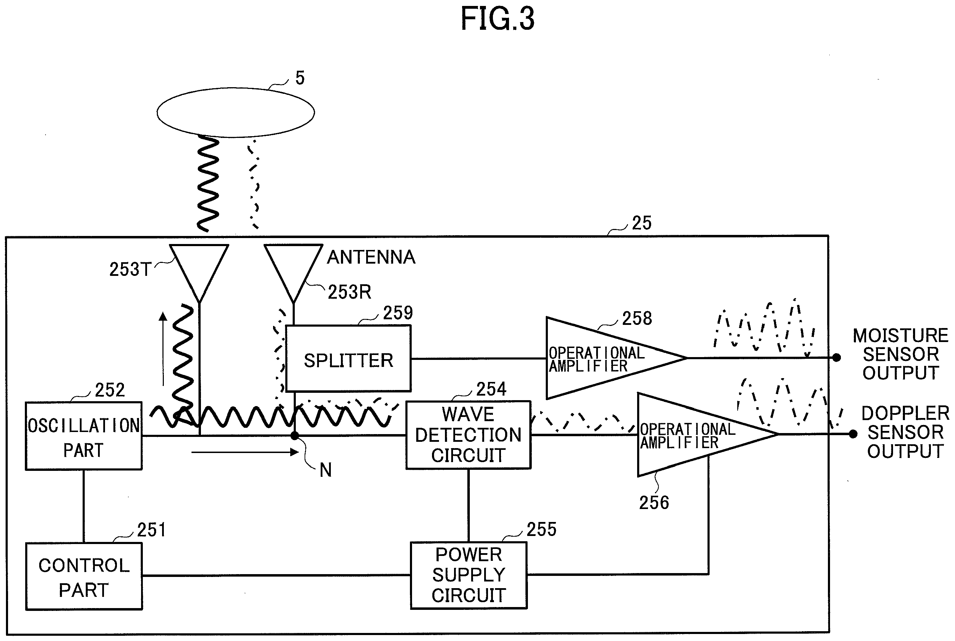

Fig. 3 is a block diagram for illustrating an example of the radio wave transmission/reception part 25.

The radio wave transmission/reception part 25 includes a control part 251, an oscillation part 252, antennas 253T, 253R, a wave detection circuit 254, a power supply circuit 255, and operational amplifiers 256, 258. Transmission waves (radio waves) generated in the oscillation part 252 are divided to be supplied to the antenna 253T and the wave detection circuit 254, and the waves transmitted from the antenna 253T impinge on the test subject 5. The waves impinging on the test subject 5 are reflected, and the reflection waves from the test subject 5 are received by the antenna 253R. The reflection waves received by the antenna 253R and indicated by alternate long and short dashed lines are divided at a splitter 259 to be supplied to a node N and the operational amplifier 258. The reflection waves supplied to the node N interferes with the transmission waves indicated by a solid line, and superposition waves (DC components) indicated by alternate long and short dashed lines are output from the wave detection circuit 254. The operational amplifier 256 inputs the amplified superposition waves to the CPU 11 via the communication interface 17 as a sensor output. The sensor output from the amplifier 256 is referred to as a "Doppler sensor output". The operational amplifier 258 inputs the amplified reflection waves to the CPU 11 via the communication interface 17 as a sensor output. The sensor output from the amplifier 258 is referred to as a "moisture sensor output".

The power supply circuit 255 includes a battery that supplies a supply voltage to the control part 251, the oscillation part 252, the wave detection circuit 254, and the operational amplifier 256. The battery is rechargeable, for example. It is noted that the power supply circuit 255 may be located outside of the radio wave transmission/reception part 25 and coupled to the radio wave transmission/reception part 25. Further, the antennas 253T, 253R may be integrated as a transmission/reception antenna.

It is noted that, in the example illustrated in Fig. 3, the radio wave transmission part 1 includes at least the oscillation part 252 and the antenna 253T, and the radio wave reception part 2 includes at least the antenna 253R and the operational amplifier 258. It is noted that, in the example illustrated in Fig. 3, the radio wave transmission/reception part 25 includes a configuration that generates the Doppler sensor output; however, the configuration that generates the Doppler sensor output may be omitted.

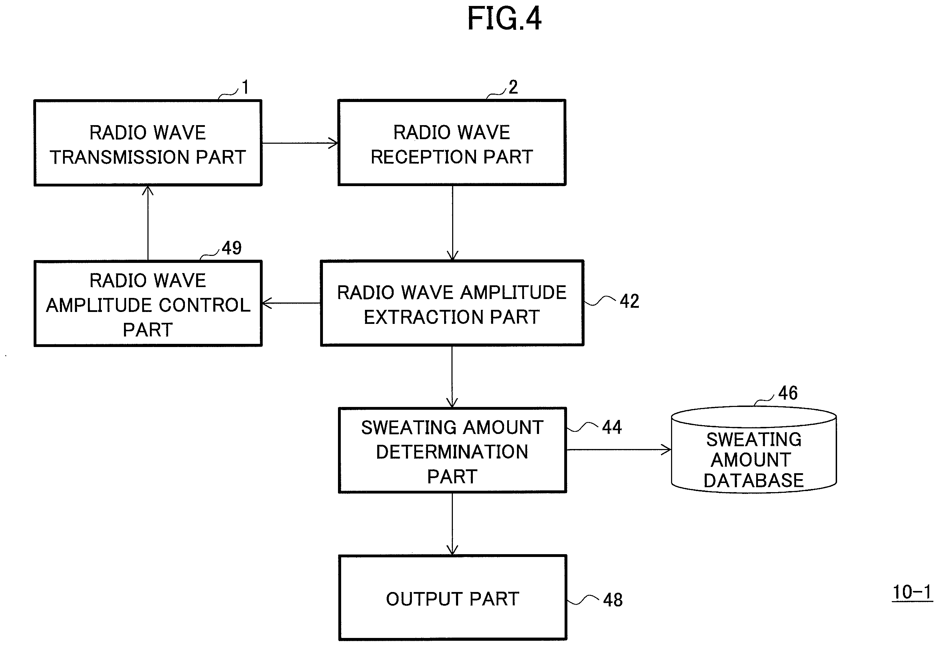

Fig. 4 is a block diagram illustrating an example of a functional configuration of the state determination system 10-1.

In the example illustrated in Fig. 4, the state determination system 10-1 includes the radio wave transmission part 1, the radio wave reception part 2, a radio wave amplitude extraction part 42, a sweating amount determination part 44, a sweating amount database 46, an output part 48, and a radio wave amplitude control part 49. In the example illustrated in Fig. 4, the radio wave transmission part 1 and the radio wave reception part 2 can be implemented by the radio wave transmission/reception part 25 illustrated in Fig. 2. Further, the functions of the radio wave amplitude extraction part 42, the sweating amount determination part 44, the output part 48, and the radio wave amplitude control part 49 can be implemented by the CPU 11 illustrated in Fig. 2. The sweating amount database 46 can be implemented by the ROM 13 illustrated in Fig. 2. Other auxiliary storage devices such as a HDD (Hard Disk Drive), etc., may be used instead of the ROM 13.

The radio wave amplitude extraction part 42 calculates the amplitude of signal of the reflection waves (i.e., the moisture sensor output) input from the operational amplifier 258 of the radio wave reception part 2. The amplitude is an index representing a strength of the signal of the reflection waves. The amplitude may be determined from one peak to a next peak of the waveform of the signal of the reflection waves, or may be determined on a peak basis. Further, the radio wave amplitude extraction part 42 may output an average value of the calculated values of the amplitude over a predetermined time period.

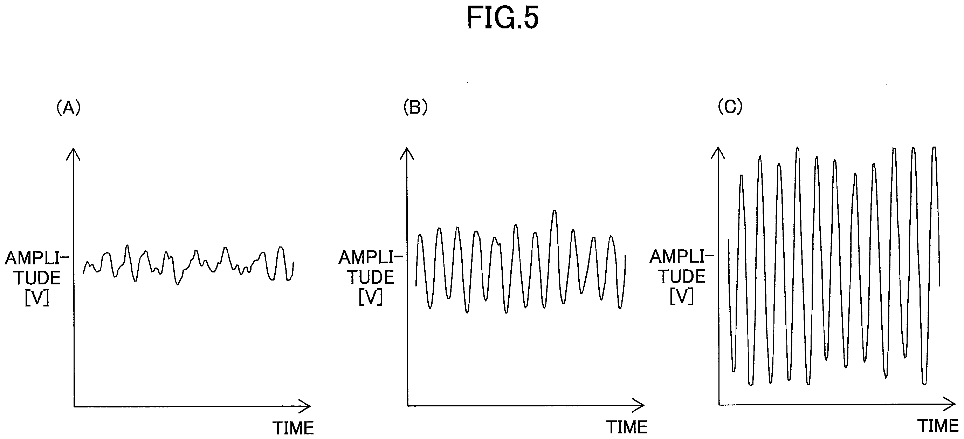

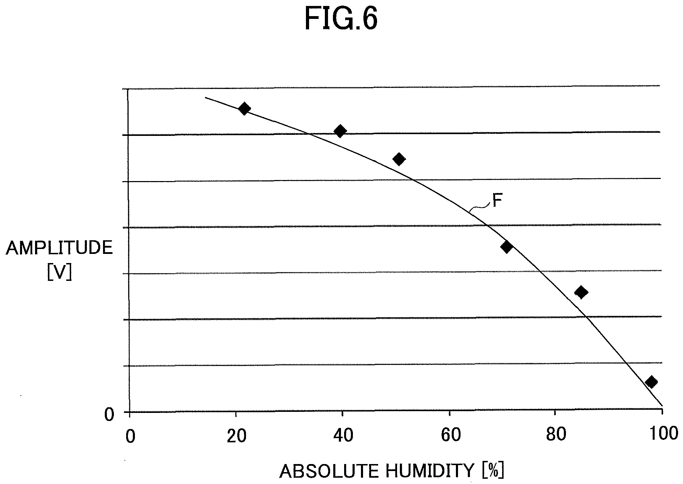

The sweating amount determination part 44 determines a moisture state of the human body of the test subject 5 or a moisture state of the wear item 6 based on the amplitude obtained from the radio wave amplitude extraction part 42. Here, the radio waves transmitted from the radio wave transmission part 1 are attenuated at the time of the reflection at the human body, and at the time of passing through the wear item 6 before the reflection waves thereof are received by the radio wave reception part 2. Specifically, when the radio waves impinge on the human body, the radio waves are absorbed by skin, muscle, fat, bone, etc. If the skin includes a large amount of the moisture content, the absorption amount becomes greater, and thus the reflection amount of the radio waves is attenuated. Thus, if the test subject 5 sweats due to the sports, etc., to have a film of the water on a surface of the skin due to the sweat (the same holds true for the water of the soaked wear item 6), the strength of the reflection signal is attenuated. For example, the radio waves in the 2.4 GHz band have the attenuation 0.4 dB at the water film of 0.1mm thickness, and the radio waves in the 24 GHz band have the attenuation 6 dB at the water film of 0.1mm thickness. In this way, the inventor of the present invention confirms that there is a correlation between the attenuation amount of the radio waves and the moisture state of the human body or the moisture state of the wear item 6 (see Fig. 5 and Fig. 6). The attenuation amount of the radio waves corresponds to the reduced amount of the amplitude. Fig. 5 is a diagram illustrating a relationship between absolute humidity and an amplitude of a waveform of a Doppler sensor output. In Fig. 5, (A) illustrates the case of the absolute humidity being 85%, (B) illustrates the case of the absolute humidity being 50%, and (C) illustrates the case of the absolute humidity being 20%. In Fig. 5, a horizontal axis represents a time, and a vertical axis represents an amplitude. Fig. 6 illustrates the amplitude obtained by the radio wave amplitude extraction part 42 in the vertical axis. In Fig. 6, the horizontal axis represents the absolute humidity. It is noted that, in Fig. 5, the waveform of the Doppler output is illustrated; however, substantially the same trend can be seen in the waveform of the signal of the reflection waves (i.e., the moisture sensor output). Further, in Fig. 5 and Fig. 6, for the sake of explanation, the amplitude is expressed by the voltage value [V] of the signal of the reflection waves. The absolute humidity is at the space between the human body and the wear item 6, and is an index representing the moisture state of the human body and the moisture state of the wear item 6. As illustrated in Fig. 5 and Fig. 6, the amplitude of the reflection waves becomes smaller as the absolute humidity becomes higher. In other words, the attenuation amount becomes greater as the absolute humidity becomes higher. The amplitude of the reflection waves and the absolute humidity can be associated with each other by a certain relationship F. The relationship F can be derived in advance with a curve fitting using a plurality of items of actual data, as illustrated in Fig. 6. The relationship F may be stored in the sweating amount database 46.

The sweating amount determination part 44 determines a sweating amount of the human body (an example of a sweating state) based on the determined moisture state of the human body and the determined moisture state of the wear item 6. There is a correlation between the moisture state of the human body or the moisture state of the wear item 6 and the sweating amount. Basically, the moisture state of the human body or the moisture state of the wear item 6 increases as the sweating amount increases. The moisture state of the human body or the moisture state of the wear item 6 and the sweating amount can be associated with each other by a certain relationship F1. The relationship F1 may be a proportional relationship (a linear function), for example. The relationship F1 may be stored in the sweating amount database 46.

However, the sweating amount determination part 44 may determine the sweating amount of the human body directly based on the amplitude obtained by the radio wave amplitude extraction part 42. Specifically, the sweating amount determination part 44 may determine the sweating amount of the human body directly based on the amplitude obtained by the radio wave amplitude extraction part 42 without determining the moisture state of the human body or the moisture state of the wear item 6. This is because there is a correlation between the amplitude of the reflection waves and the sweating amount of the human body, which is because of a fact that there is a correlation between the amplitude of the reflection waves and the moisture state of the human body or the moisture state of the wear item 6 and there is a correlation between the moisture state of the human body or the moisture state of the wear item 6 and the sweating amount of the human body.

The sweating amount database 46 stores the relationship F that associates the amplitude of the reflection waves with the absolute humidity. Alternatively, the sweating amount database 46 may store table data of many associated pairs of the amplitude of the reflection waves and the absolute humidity. Further, the sweating amount database 46 stores the relationship F1 that associates the sweating amount with the absolute humidity. Alternatively, the sweating amount database 46 may store table data of many associated pairs of the absolute humidity and the sweating amount.

Alternatively, the sweating amount database 46 may store the relationship F2 that associates the amplitude of the reflection waves with the sweating amount. Alternatively, the sweating amount database 46 may store table data of many associated pairs of the amplitude of the reflection waves and the sweating amount.

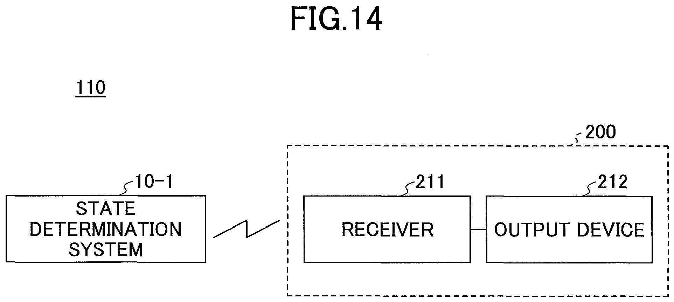

The output part 48 outputs the sweating amount determined by the sweating amount determination part 44. A way of outputting the sweating amount determined by the sweating amount determination part 44 is arbitrary. For example, the sweating amount determined by the sweating amount determination part 44 may be outputted on the display device 22, or may be outputted with sound messages, etc. Further, the sweating amount determined by the sweating amount determination part 44 may be transmitted to an external device (a center 200 illustrated in Fig. 14, for example) via the wireless communication by the wireless transmission/reception part 26. In this case, for example, when the sweating amount determined by the sweating amount determination part 44 exceeds a predetermined threshold, the output part 48 may transmit information, which represents that the determined sweating amount exceeds the predetermined threshold, to the external device. Other examples of the operation of the output part 48 are described hereinafter.

The radio wave amplitude control part 49 controls transmission power of the radio wave transmission part 1 based on the amplitude obtained by the radio wave amplitude extraction part 42 such that the amplitude becomes maximum. The control (adjustment) of the transmission power is performed once in an initial state (in a state in which the sweating amount of the human body is substantially 0, for example). In this way, by initially maximizing the amplitude of the reflection waves, it becomes possible to increase a resolution for evaluating the attenuation amount and thus increase the accuracy of the determined sweating amount. However, the radio wave amplitude control part 49 is not indispensable, and may be omitted. In this case, the transmission power of the radio wave transmission part 1 is constant.

According to the state determination system 10-1 illustrated in Fig. 4, it becomes possible to determine the moisture state of the human body or the moisture state of the wear item 6 of the test subject 5 based on the moisture sensor output. The moisture sensor output is generated by the radio wave transmission part 1 and the radio wave reception part 2, as described above. Thus, according to the state determination system 10-1 illustrated in Fig. 4, it becomes possible to determine the moisture state of the human body or the moisture state of the wear item 6 of the test subject 5 utilizing the radio waves. Further, according to the state determination system 10-1 illustrated in Fig. 4, it becomes possible to determine the sweating amount of the test subject 5 based on the determined moisture state of the human body or the moisture state of the wear item 6 of the test subject 5. Thus, according to the state determination system 10-1 illustrated in Fig. 4, it becomes possible to determine the sweating amount of the test subject 5 utilizing the radio waves.

Further, according to the state determination system 10-1 illustrated in Fig. 4, since the radio waves are utilized, the moisture state of the human body or the moisture state of the wear item 6 and the sweating amount (referred to as "the moisture state of the human body or the moisture state of the wear item 6, etc." hereinafter) can be determined in a non-contact way. Unlike the light, the radio waves are not influenced by the color of the reflection surface (i.e., the skin) of the human body. Thus, according to the state determination system 10-1 illustrated in Fig. 4, it becomes possible to determine the moisture state of the human body or the moisture state of the wear item 6, etc., even if the radio wave impinging location cannot be fixed in the human body due to the motion of the test subject 5, etc. Further, in the case of the measurement with the light, the light absorption factor varies according to the material of the wear item 6, and thus the amount of the reflected light varies according to the material of the wear item 6. In contrast, with respect to the light, the radio waves have the decreased change in the strength of the reflection waves due to the wear item 6. Thus, according to the state determination system 10-1 illustrated in Fig. 4, it becomes possible to determine the moisture state of the human body or the moisture state of the wear item 6 of the test subject 5, etc., regardless of the material of the wear item 6 on the test subject 5. Further, in the case of the measurement with the light, when the sweat, the oil or the like adheres to the light emitting part or the light reception part, the amount of the reflection light is reduced. In contrast, in the case of the radio waves, even if the sweat, the oil or the like adheres to the radio wave transmission part 1 or the radio wave reception part 2, the reduced amount of the strength of the reflection waves is smaller with respect to the light. Thus, according to the state determination system 10-1 illustrated in Fig. 4, it becomes possible to determine the moisture state of the human body or the moisture state of the wear item 6 of the test subject 5, etc., even in a circumstance where the sweat, the oil or the like easily adheres thereto.

Further, according to the state determination system 10-1 illustrated in Fig. 4, the moisture state of the human body or the moisture state of the wear item 6 is determined utilizing a fact that there is a correlation between the attenuation amount of the radio waves and the moisture state of the human body or the moisture state of the wear item 6. Thus, according to the state determination system 10-1 illustrated in Fig. 4, it becomes possible to determine the moisture state of the human body or the moisture state of the wear item 6 with the increased accuracy. This holds true for the determination of the sweating amount.

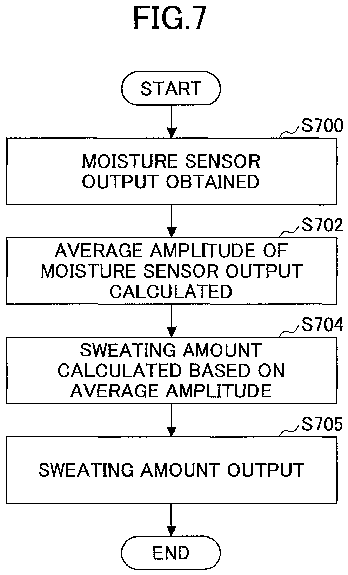

Fig. 7 is a flowchart illustrating an example of the state determination process executed by the CPU 11 of the state determination system 10-1. A process routine illustrated in Fig. 7 may be executed repeatedly at a predetermined cycle.

In step S700, the radio wave amplitude extraction part 42 obtains the moisture sensor output (the signal of the reflection waves input from the operational amplifier 258 of the radio wave reception part 2) over a time period ΔT (1 sec, for example) corresponding to the predetermined cycle.

In step S702, the radio wave amplitude extraction part 42 calculates the average value of the amplitude of the moisture sensor output over the time period ΔT based on the moisture sensor output over the time period ΔT.

In step S704, the sweating amount determination part 44 calculates the sweating amount based on the amplitude (i.e., the average value) calculated in step S702. In this example, the sweating amount database 46 may store the relationship F2 that associates the amplitude of the reflection waves with the sweating amount. The sweating amount determination part 44 obtains the sweating amount by substituting the amplitude calculated in step S702 into the relationship F2.

In step S705, the output part 48 outputs the calculation result of the sweating amount obtained in step S704. A destination to which the calculation result of the sweating amount is to be outputted is arbitrary. The destination may be the center 200 (see Fig. 14) that monitors the test subject 5, etc., for example.

According to the state determination process illustrated in Fig. 7, the sweating amount determination part 44 can determine the sweating amount of the test subject 5 based on the moisture sensor output over the time period ΔT. Further, the output part 48 can output the determination result of the sweating amount of the test subject 5. The moisture sensor output is generated by the radio wave transmission part 1 and the radio wave reception part 2, as described above. Thus, according to the state determination process illustrated in Fig. 7, it becomes possible to determine and output the sweating amount of the test subject 5 utilizing the radio waves. Further, by utilizing the radio waves, the same effects as described above with reference to Fig. 4 can be obtained.

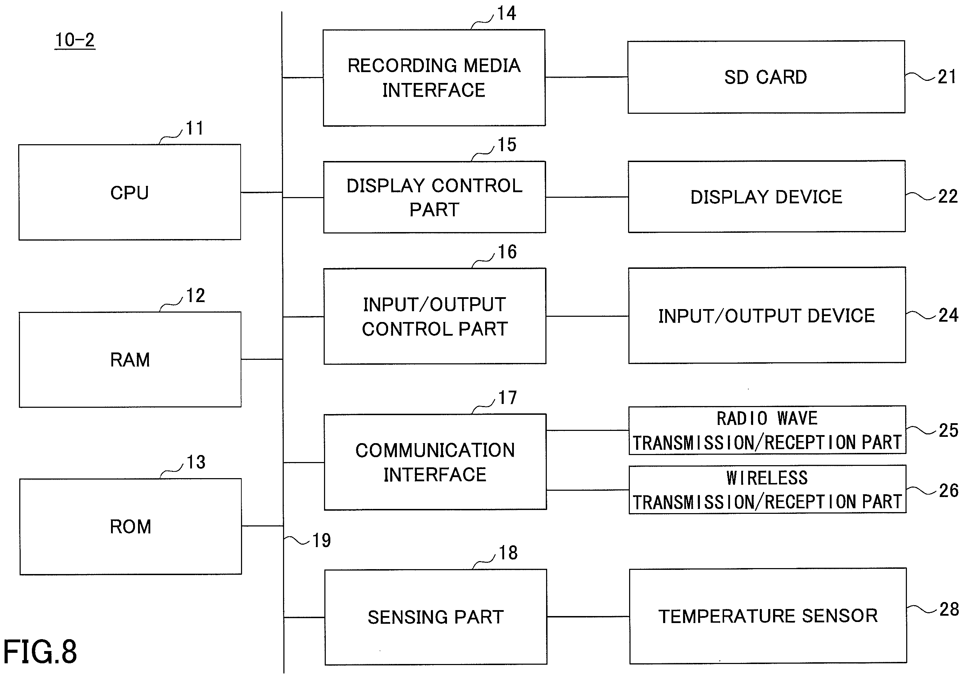

Fig. 8 is a block diagram illustrating an example of a hardware configuration of a state determination system 10-2 according to another embodiment. In Fig. 8, elements that may be the same as those illustrated in Fig. 2 are given the same reference numerals, and explanation thereof is omitted.

The hardware configuration of the state determination system 10-2 differs from that of the state determination system 10-1 illustrated in Fig. 2 in that a sensing part 18 is added.

The sensing part 18 is coupled to a temperature sensor 28. The temperature sensor 28 detects an ambient temperature. The temperature sensor 28 is attached to the test subject 5, for example. The temperature sensor 28 may be directly attached to the test subject 5; however, typically, the temperature sensor 28 is attached to the wear item 6 or another wear item on the test subject 5. It is noted that the state determination system 10-2 is attached to the hardness, the temperature sensor 28 detects the temperature around the hardness.

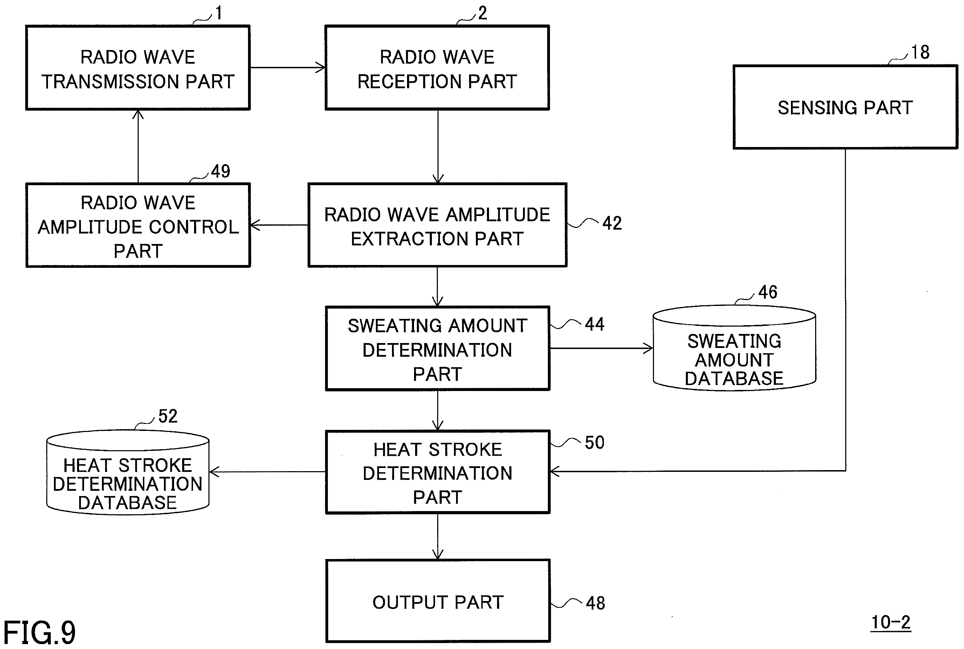

Fig. 9 is a block diagram illustrating an example of a functional configuration of the state determination system 10-2. In Fig. 9, elements that may be the same as those illustrated in Fig. 4 are given the same reference numerals, and explanation thereof is omitted.

In the example illustrated in Fig. 9, the state determination system 10-2 includes the radio wave transmission part 1, the sensing part 18, the radio wave reception part 2, the radio wave amplitude extraction part 42, the sweating amount determination part 44, the sweating amount database 46, the output part 48, the radio wave amplitude control part 49, a heat stroke determination part 50, and a heat stroke determination database 52. In the example illustrated in Fig. 9, the radio wave transmission part 1 and the radio wave reception part 2 can be implemented by the radio wave transmission/reception part 25 illustrated in Fig. 8. Further, the functions of the radio wave amplitude extraction part 42, the sweating amount determination part 44, the output part 48, the radio wave amplitude control part 49, and the heat stroke determination part 50 can be implemented by the CPU 11 illustrated in Fig. 8. The sweating amount database 46 and the heat stroke determination database 52 can be implemented by the ROM 13 illustrated in Fig. 2. Other auxiliary storage devices such as a HDD, etc., may be used instead of the ROM 13.

The heat stroke determination part 50 refers to the heat stroke determination database 52 to determine a hazard level of the heat stroke (an example of a determination of a state of the human body) based on the sweating amount determined by the sweating amount determination part 44 and the temperature information (the detection result of the temperature sensor 28, for example) from the sensing part 18.

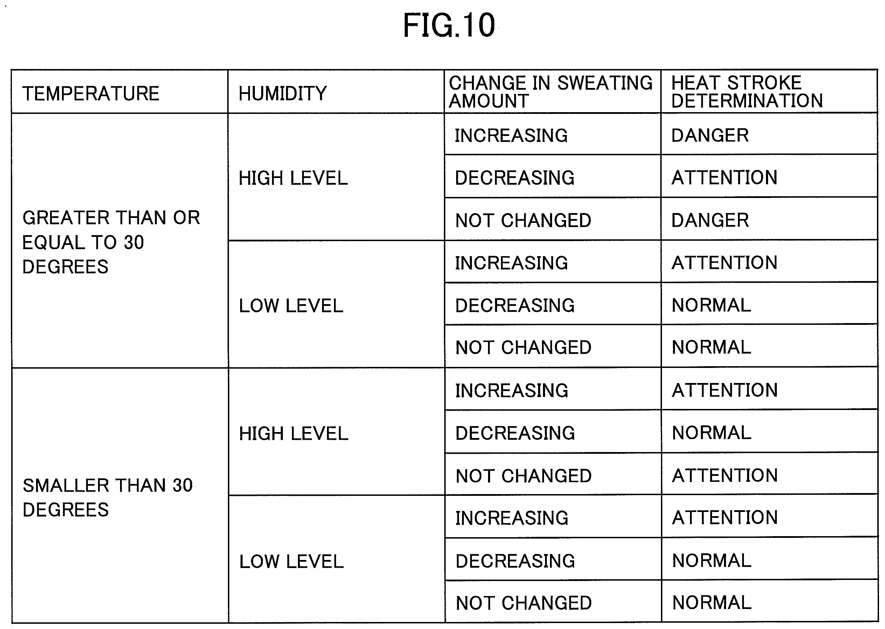

In the heat stroke determination database 52, a determination logic for the hazard level of the heat stroke as illustrated in Fig. 10 is stored. In the example illustrated in Fig. 10, a temperature condition is divided into being greater than or equal to 30 degrees and being less than 30 degrees. Further, a humidity condition is divided into a high level and a low level. The high level and the low level may be distinguished by arbitrary references. Further, a sweating amount condition is divided into "increasing", "decreasing", and "not changed". The determination results of the heat stroke according to combinations of the respective conditions are illustrated. For example, if the temperature is greater than or equal to 30 degrees, the humidity is at high level, and the sweating amount increasing, the determination result of the heat stroke is "danger". The "danger" represents a very high probability of a transition into the heat stroke, and may include a state of the actual heat stroke. On the other hand, if the temperature is less than 30 degrees, the humidity is at high level, and the sweating amount increasing, the determination result of the heat stroke is "attention". It is noted that the "attention" represents the reduced probability of the transition into the heat stroke with respect to the "danger".

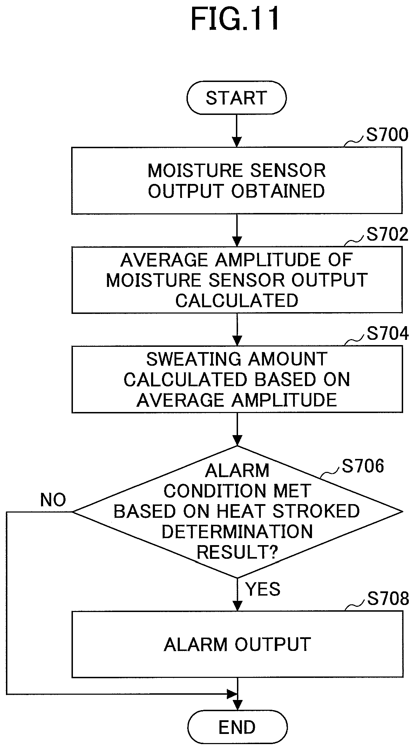

Fig. 11 is a flowchart illustrating an example of a state determination process executed by the CPU 11 of the state determination system 10-2. A process routine illustrated in Fig. 11 may be executed repeatedly at a predetermined cycle.

The process illustrated in Fig. 11 differs from the process illustrated in Fig. 7 in that the process of step S705 is omitted and processes after step S706 are added. In the following, the difference is described.

In step S706, the output part 48 refers to the heat stroke determination database 52 to obtain the heat stroke determination result based on a history (an example of a change in the determination result in a time series) of the calculate results of the sweating amount obtained in step S704 and the temperature information from the sensing part 18. The output part 48 determines, based on the heat stroke determination result, whether an alarm condition is met. The alarm condition is arbitrary. The alarm condition may be met when the heat stroke determination result is "danger", or may be met when the heat stroke determination result is "danger" or "attention". For example, the alarm condition may be met upon a state in which the sweating amount is not changed (a state in which the change amount is smaller than a reference) after a state in which the sweating amount has increased. This is because the state in which the sweating amount is not changed (i.e., the sweat is not evaporated) after the sweating amount has increased means a state in which a body temperature cannot be reduced by heat of evaporation of the sweat, that is to say, a state in which the transition to the heat stroke state is highly anticipated. If it is determined that the alarm condition is met, the process routine goes to step S708, otherwise the process routine at the current cycle directly ends.

In step S708, the output part 48 outputs an alarm (an example of information representing hazard of the human body). A destination to which the alarm is to be outputted is arbitrary. The destination may be the center 200 (see Fig. 14) that monitors the test subject 5, etc., for example. A way of outputting the alarm may be varied according to the heat stroke determination result. It is noted that the output part 48 may output information indicating "normal" even when the heat stroke determination result is "normal". In this case, the output part 48 may output the heat stroke determination result together with the alarm when the heat stroke determination result changes from "normal" to "danger" or "attention".

According to the state determination process illustrated in Fig. 11, the alarm can be outputted when it is determined, based the history (a change in a time series) of the calculation result of the sweating amount, etc., that the alarm condition related to the heat stroke is met. With this arrangement, during the training session or sports session of the test subject 5, for example, upon a detection of a state in which the test subject 5 may be subject to the heat stroke with a high probability, such a fact can be reported to a supervisor of the test subject 5, for example. As a result of this, the safety of the test subject 5 can be enhanced. For example, the state determination system 10-2 can function of monitoring a human body state of the troop (i.e., the test subject 5) wearing protective clothing, for example.

It is noted that the determination logic (the example illustrated in Fig. 10) used in the state determination process illustrated in Fig. 11 is just an example, and can be changed variously to match the actual cases of the heat stroke. For example, the temperature condition and/or the humidity condition and/or the sweating amount condition may be divided more.

Further, as a preferable embodiment, for example, the content of the alarm may be varied between the case in which the determination result indicates a non-changed state of the sweating amount after indicating the sweating amount increasing state and the case in which the determination result indicates the sweating amount decreasing state after indicating the sweating amount increasing state. This is because the hazard level is lower in the case in which the sweating amount decreases (i.e., the sweat evaporates) after having sweated (i.e., the sweating amount has increased) than in the case in which the sweating amount does not change. The content of the alarm may be varied such that the attention attraction capability (the volume level of an alarm buzzer, for example) becomes greater as the hazard level becomes greater, for example. In this way, the safety of the test subject 5 can be enhanced by varying the content of the alarm according to the presence or absence of the decrease in the sweating amount after the increase in the sweating amount.

Further, the state determination system 10-2 determines whether the alarm condition related to the heat stroke is met; however, in addition to or instead of the condition, other conditions may be determined. In other words, the alarm condition is arbitrary, and may be set according to the monitoring purpose (the heat stroke prevention, a heart abnormality monitoring, a drip accident monitoring, a diaper exchange timing monitoring, etc., for example), if appropriate. With this arrangement, when an abnormal sweating amount of the test subject 5 (a large amount of cold sweat at the time of the heart abnormality) is detected, for example, such a fact can be reported to the supervisor of the test subject 5. Further, if the test subject 5 is a patient subject to the drip, the drip accident, in which the drip is removed accidentally, can be detected based on the sudden increase in the moisture state of the wear item 6 due to the drip accident. Also, in this case, the fact can be reported to the supervisor of the test subject 5, for example. It is noted that, in the case of the drip accident monitoring or the diaper exchange timing monitoring, the sweating amount determination part 44 need not calculate the sweating amount. For example, in the case of the diaper exchange timing monitoring, the sweating amount determination part 44 may determine the moisture state of the diaper (an example of the wear item 6) to determine, based on the determine moisture state, whether the alarm condition is met (or determine the care timing). The alarm condition may be met when the determined moisture state indicates the moisture exceeding a predetermined moisture reference, for example. With this arrangement, the output part 48 can output, as the alarm, the report related to an occurrence of a wetted state of the diaper, and /or related to the exchange of the diaper In this way, the state determination system 10-2 can function of monitoring the state of a care subject (the test subject 5) in a facility such as a nursing care facility, a hospital, etc.

Fig. 12 is a diagram illustrating an example of a way of holding a radio wave transmission/reception part 25.

In the example illustrated in Fig. 12, the radio wave transmission/reception part 25 (an example of a sensor) is held in a holder 8 of a hardness 7. The hardness 7 is attached to the outside of the wear item 6 (an example of a first wear item). The holder 8 is provided on an outer surface (opposite to the side opposed to the wear item 6) of the hardness 7, for example. The radio wave transmission/reception part 25 thus provided in such a positional relationship transmits the radio waves to the human body of the test subject 5, who wears the wear item 6 and the hardness 7, via the wear item 6 and the hardness 7. The holder 8 may be in any forms. The holder 8 may be in form of a pocket, a belt, Magic Tape (registered trademark), Velcro (registered trademark), etc.

Fig. 13 is a diagram schematically illustrating an example of a hardness 7.

In the example illustrated in Fig. 13, the hardness 7 includes the holder 8 on the inner side (i.e., the side opposed to the human body). The hardness 7 may include pockets for accommodating a water bottle, other sensor devices, a wireless communication device, etc., in addition to the radio wave transmission/reception part 25. The hardness 7 may be in form of a belt or the like, or in form of a vest.

Fig. 14 is a diagram illustrating an example of a configuration of a monitoring system 110.

The monitoring system 110 includes the state determination system 10-1 and a center 200.

The state determination system 10-1 may be as described above, but includes the wireless transmission/reception part 26 in this example. Further, with respect to the state determination system 10-1 included in the monitoring system 110, the display device 22 and the display control part 15 may be omitted, and the recording media interface 14 and the SD card 21, the input/output control part 16 and the input/output device 24 may also be omitted. In the example, the radio wave transmission/reception part 25 (an example of a sensor) of the state determination system 10-1 is accommodated in the holder 8 of the hardness 7 (see Fig. 12 and Fig. 13, for example). Other elements of the state determination system 10-1 may be accommodated in the holder 8 of the hardness 7 as is the case with the radio wave transmission/reception part 25.

The wireless transmission/reception part 26 of the state determination system 10-1 transmits the information representing the moisture state of the human body or the moisture state of the wear item 6 of the test subject 5 and/or the information representing the sweating amount of the test subject 5, which can be obtained as described above, to the center 200. For example, the wireless transmission/reception part 26 transmits the information outputted by the output part 48 to the center 200.

The center 200 is provided remotely with respect to the holder 8 of the hardness 7. The center 200 may be a monitoring center that monitors trainers who are subject to the training and wear the harnesses 7, for example. The center 200 may be in the form of a server.

The center 200 includes a receiver 211 and an output device 212, as illustrated in Fig. 14.

The receiver 211 receives the information (i.e., the information representing the moisture state of the human body or the moisture state of the wear item 6 of the wear item 6, etc.) transmitted from the wireless transmission/reception part 26.

The output device 212 outputs the information received by the receiver 211. A way of outputting the information received by the receiver 211 is arbitrary. For example, the information received by the receiver 211 may be outputted on a display apparatus (not illustrated) in real-time. The output device 212 may output the information on a test subject 5 basis, when the information related to a plurality of the test subjects 5 is received from the respective state determination systems 10-1.

It is noted that, in the example illustrated in Fig. 14, the wireless transmission/reception part 26 of the state determination system 10-1 transmits the information representing the moisture state of the human body or the moisture state of the wear item 6 of the test subject 5 and/or the information representing the sweating amount of the test subject 5 to the center 200; however, this is not indispensable. For example, the wireless transmission/reception part 26 may transmit the moisture sensor output itself to the center 200. In this case, the center 200 may include functional parts corresponding to the radio wave amplitude extraction part 42, the sweating amount determination part 44, the sweating amount database 46, and the output part 48 of the state determination system 10-1 (see Fig. 4). Alternatively, the wireless transmission/reception part 26 may transmit the information obtained by the radio wave amplitude extraction part 42 to the center 200. In this case, the center 200 may include functional parts corresponding to the sweating amount determination part 44, the sweating amount database 46, and the output part 48 of the state determination system 10-1 (see Fig. 4).

Further, in the example illustrated in Fig. 14, the monitoring system 110 includes the state determination system 10-1; however, the monitoring system 110 may include the state determination system 10-2, instead of the state determination system 10-1. In this case, the wireless transmission/reception part 26 may transmit the heat stroke determination result to the center 200, or may transmit data (i.e., information obtained by the temperature sensor 28, etc.), which can be used to obtain the heat stroke determination result, to the center 200.

All examples and conditional language recited herein are intended for pedagogical purposes to aid the reader in understanding the invention and the concepts contributed by the inventor to furthering the art, and are to be construed as being without limitation to such specifically recited examples and conditions, nor does the organization of such examples in the specification relate to a showing of the superiority and inferiority of the invention. Although the embodiment(s) of the present inventions have been described in detail, it should be understood that the various changes, substitutions, and alterations could be made hereto without departing from the spirit and scope of the invention. Further, all or part of the components of the embodiments described above can be combined.

For example, in the embodiments described above, the sweating amount determination part 44 determines the moisture state of the human body or the moisture state of the wear item 6, etc., based the amplitude of the reflection waves, using the information representing the correlation between the amplitude of the reflection waves and the absolute humidity. However, as an equivalent variant, the sweating amount determination part 44 may determine the moisture state of the human body or the moisture state of the wear item 6, etc., based the attenuated amount of the amplitude of the reflection waves, using information representing the correlation between the attenuated amount of the amplitude of the reflection waves and the absolute humidity. In this case, the attenuated amount of the amplitude of the reflection waves may be calculated with respect to a reference value of the amplitude of the reflection waves. The reference value of the amplitude of the reflection waves may be the amplitude of the reflection waves obtained in the initial state (in a state in which the sweating amount of the human body is substantially 0, for example). Further, information representing the correlation between the amplitude of the reflection waves and a relative humidity or an impedance, instead of the absolute humidity, may be used. For example, the sweating amount determination part 44 may determine the moisture state of the human body or the moisture state of the wear item 6, etc., based on information representing the correlation between the amplitude of the reflection waves and the relative humidity, and the temperature information from the temperature sensor 28.

Further, in the embodiments described above, the sweating amount determination part 44 determines the moisture state of the human body or the moisture state of the wear item 6, etc., based on the moisture sensor output; however, the sweating amount determination part 44 may determine the moisture state of the human body or the moisture state of the wear item 6, etc., based on the Doppler sensor output, which can be understood from Fig. 5. In this case, in the configuration illustrated in Fig. 3, the splitter 259 and the operational amplifier 258 may be omitted. The sweating amount determination part 44 may calculate the amplitude of the waveform of the Doppler sensor output, or may calculate the amplitude (a peak value at a particular frequency) of the reflection waves based on a frequency analysis result obtained by a FFT (Fast Fourier Transform) process of the Doppler sensor output.

### [Description of Reference Symbols]

- 1: radio wave transmission part
- 2: radio wave reception part
- 6: wear item
- 10-1, 10-2: state determination system
- 25: radio wave transmission/reception part
- 28: temperature sensor
- 200: center
- 110: monitoring system
- 211: receiver
- 212: output device

## Claims

1. A method of determining a state, the method being to be executed by a computer, said method comprising:
determining a moisture state according to a reception state of a reflection wave of a radio wave, the radio wave being transmitted to a human body via a wear item, the wear item being on the human body, and
outputting according to the determined result.

2. The method of claim 1, wherein the wear item is on an upper-body side of the human body, and an output according to the determined result includes information representing a sweating state of the human body.

3. The method of claim 1, wherein the wear item is a diaper, and further comprising reporting an occurrence of a wetted state of the diaper or a report related to an exchange of the diaper when the determined result indicates a moisture greater than a predetermined moisture reference.

4. The method of claim 1, further comprising determining a state of the human body according to a change in the determined moisture state in a time series.

5. The method of claim 1, wherein the output includes information representing a hazard to the human body, the information being outputted based on the determined result indicating a detection of an event, the event being that a change in a moisture level is smaller than a reference after an increase in the moisture level.

6. The method of any one of claims 1 through 5, wherein the radio wave has a frequency in UHF band or in SHF band.

7. The method of claim 1, further comprising changing a way of outputting according to the determined result based on a combination of a change in the determined result and a determination whether a temperature is greater than or equal to a predetermined value.

8. The method of claim 1, wherein the moisture state is a moisture state of the wear item, a moisture state of the human body, or a sweating state of the human body.

9. The method of claim 1, wherein the reception state of the reflection wave includes a strength of the reflection wave.

10. The method of claim 9, wherein the strength of the reflection wave includes an amplitude of a waveform of the reflection wave from one peak to another peak.

11. The method of claim 1, wherein determining the moisture state includes calculating a strength of the reflection wave, and calculating the moisture state based on a calculation result of the strength of the reflection wave and information, the information associating the strength of the reflection wave with the moisture state.

12. The method of claim 10, wherein the information associating the strength of the reflection wave with the moisture state includes information representing a relationship between the moisture state and an attenuation amount of the strength of the reflection wave with respect to a predetermined reference value.

13. The method of claim 4, further comprising determining, based on a change in the determined moisture state in a time series, whether to output according to the determined result.

14. The method of claim 4, wherein determining the state of the human body includes a determination related to a probability of a heat stroke.

15. The method of claim 7, further comprising detecting the temperature with a temperature sensor attached to the human body.

16. A state determination system, comprising:
a determination part configured to determine a moisture state according to a reception state of a reflection wave of a radio wave, the radio wave being transmitted to a human body via a wear item, the wear item being on the human body, and
an output part configured to output according to the determined result.

17. The state determination system of claim 16, further comprising:
a transmitter configured to transmit the radio wave to the human body wearing the wear item; and
a receiver configured to receive the reflection wave of the radio wave transmitted by the transmitter, wherein
the determination part determines the moisture state according to a reception state of the reflection wave received by the receiver.

18. A wear item to be on a human body and on an outer side of a first wear item, the wear item comprising:
a holder configured to hold a sensor, the sensor being configured to transmit a radio wave to a human body side via the first wear item on the human body, and receive a reflection wave of the radio wave.

19. The wear item of claim 18, wherein the wear item is a hardness.

20. The wear item of claim 18 or 19, wherein the holder is on a side the wear item opposite to a side opposed to the first wear item.

21. A monitoring system, comprising:
a sensor configured to be held in a wear item, the wear item being configured to be on a human body and on an outer side of a first wear item, the sensor being further configured to transmit a radio wave to the human body side via the first wear item, and receive a reflection wave of the radio wave;
a transmission device configured to transmit information representing a reception state of the reflection wave received by the sensor or information representing a moisture level, the moisture level being obtained by analyzing the reception state;
a reception device configured to receive the information transmitted by the transmission device; and
an output device configured to output, based on the information received by the reception device, information related to a moisture state of the human body or a moisture state of the first wear item.
